# FASCICULE DE BREVET EUROPEEN

(11) **EP 3 749 950 B1**
(45) Date de publication et mention de la délivrance du brevet: **07.06.2023**
(21) Numéro de dépôt: 19701395.6
(22) Date de dépôt: 30.01.2019
(51) Int. Cl.: G01N 25/18, G01N 33/00

(54) **MÉTHODE DE CONTRÔLE SUR SITE DE LA QUALITÉ DES GAZ LIVRÉS SUR UN SITE INDUSTRIEL CONSOMMATEUR UTILISANT LA TECHNIQUE DE LA CONDUCTIVITÉ THERMIQUE**
KONTROLLMETHODE VOR ORT DER QUALITÄT VON GASEN, DIE AN EINEN DIESE VERBRAUCHENDEN INDUSTRIESTANDORT GELIEFERT WERDEN, MITHILFE VON WÄRMELEITFÄHIGKEIT
METHOD FOR IN-SITU MONITORING OF THE QUALITY OF GAS DELIVERED TO A CONSUMING INDUSTRIAL SITE USING THE THERMAL CONDUCTIVITY TECHNIQUE

(30) Priorité: 06.02.2018 EP 18305119
(43) Date de publication de la demande: 16.12.2020
(73) Titulaire: L'AIR LIQUIDE, SOCIETE ANONYME POUR L'ETUDE ET L'EXPLOITATION DES PROCEDES GEORGES CLAUDE, 75007 Paris (FR); Carbagas, 3073 Gümligen (CH)
(72) Inventeur: PEYER, Heinz, 3073 Gümligen (CH)
(74) Mandataire: Air Liquide
(86) Numéro de dépôt international: PCT/EP2019/052286
(87) Numéro de publication internationale: WO 2019/154697

(56) Documents cités:
- WO-A1-90/13803
- WO-A1-2014/142829
- US-A1- 2012 191 349
- US-A1- 2014 345 363
- US-A1- 2017 276 655

## Description

La présente invention concerne le domaine du contrôle de la qualité (de l' « identité ») des gaz industriels livrés sur un site industriel consommateur, tout particulièrement dans l'industrie alimentaire et pharmaceutique.

Ces industries sont notamment concernées par les gaz suivants : l'azote, l'oxygène, le CO₂, ou encore l'argon et le protoxyde d'azote, seuls ou en mélanges.

### Les documents suivants, illustrent l'état de la technique du contrôle de la qualité des gaz industriels : US2014/345363, US2017/276655, WO90/13803.

Il apparaît en effet que ces industries mettent en oeuvre de plus en plus d'exigences réglementaires, où l'acceptabilité d'une livraison de gaz sur le seul certificat d'analyse fourni par le gazier ne suffit plus (certificat qui tenait compte de tests réalisés en amont dans les propres laboratoires du gazier). Ces industries exigent maintenant la réalisation de tests de conformité (quel gaz ? quelle teneur d'un gaz dans un mélange ? etc...) sur leur site industriel, lors de la livraison (à réception).

Ces tests réalisés sur le site industriel considéré, lors de la livraison, nécessitent dès lors un temps important, obligeant le camion de livraison à attendre le résultat des tests, souvent plusieurs heures (à l'aide des méthodes classiques mentionnées dans les pharmacopées), avant d'achever sa livraison et de se diriger vers son client suivant. Ceci représente incontestablement une perte de temps très préjudiciable pour le gazier qui livre, mais aussi un cout, et une perte de temps, et de main d'oeuvre, pour l'entreprise cliente.

Un des objectifs de la présente invention est alors de proposer une nouvelle méthode de contrôle sur site de la qualité (on peut aussi dire de l' « identité ») des gaz livrés sur un site industriel consommateur.

Comme on le verra plus en détails dans ce qui suit, l'invention propose de réaliser sur le site de l'industriel livré, lors de la livraison, un contrôle du gaz ou mélange livré, par la technique de la conductivité thermique.

Selon l'invention, on dispose lors de la livraison, d'un dispositif d'identification du gaz lors de sa livraison et de sa réception sur le site consommateur, qui comprend :
- un analyseur de la conductivité thermique des gaz, analyseur qui aura été au préalable calibré à l'aide de chacun des gaz purs susceptibles d'être ultérieurement livrés, seuls ou en mélanges, durant une telle livraison ("calibration") ; et
- un système d'acquisition et de traitement de données,
dispositif d'identification du gaz qui est apte :
- à acquérir un ou des échantillons du gaz ou mélange livré ;
- à acquérir un ou des échantillons d'un gaz de référence tel l'azote, gaz de référence préféré puisqu'il est couramment présent sur de tels sites ;
- à effectuer une ou plusieurs mesures de la conductivité du ou des échantillons du gaz de référence et à effectuer la comparaison des mesures obtenues avec les valeurs de conductivité thermique obtenues en amont lors de ladite « calibration" avec ce même gaz de référence, où si lors de cette comparaison, une variation de conductivité est observée, au-delà d'une certaine déviation acceptable fixée au préalable, le dispositif est automatiquement re-calibré, tandis que si le résultat est situé dans la gamme de déviation acceptable le gaz de référence est considéré comme « reconnu » et le dispositif comme « prêt pour opération » ;
- à effectuer une ou plusieurs mesures de la conductivité du ou des échantillons du gaz ou mélange livré, et à effectuer la comparaison des mesures ainsi obtenues avec les valeurs de conductivité thermique obtenues en amont lors de ladite « calibration », où si lors de cette comparaison, le résultat est situé dans une gamme de tolérance acceptable fixée au préalable, le gaz ou mélange livré est considéré comme "reconnu" et la livraison est déclarée "conforme", tandis que si le résultat n'est pas situé dans la gamme de tolérance acceptable la livraison est déclarée « non conforme », le dispositif étant alors apte à donner un diagnostic « livraison conforme » ou « livraison non conforme » autorisant la livraison ou non du gaz ou mélange livré.

Et l'on comprend dès lors les avantages qui sont liés à cette proposition technique par rapport aux méthodes actuelles où des échantillons sont envoyés au laboratoire du site livré, où il faut attendre le retour de ce laboratoire etc... :
- cette qualification sur site lors de la livraison se fait en quelques minutes (couramment en moins de 5 minutes) ;
- chaque molécule est caractérisée par une valeur unique de conductivité thermique à une température donnée, et la littérature disponible montre bien que les conductivités thermiques de gaz tels que l'air, N₂, O₂, Ar, CO₂, N₂O, NH₃, C₂H₆, ou encore C₃H₈ sont clairement différentiables, et permettent donc d'attester que tel gaz et non tel autre est incontestablement présent dans l'échantillon testé ;

- nulle nécessité ici de disposer de gaz de calibration pour chacun des gaz ou mélanges livrés : la seule présence sur site (et elle est avérée) d'un gaz de référence tel l'azote, permet de mesurer la conductivité thermique de l'azote à titre de référence, pour confirmer le fait que l'appareillage est bien opérationnel et fournit une valeur conforme à la valeur de la conductivité de l'azote dans les gammes de température pratiquées, et que donc l'analyse de la conductivité thermique du ou des gaz d'intérêt livré(s) peut commencer ;
- aucune interférence entre gaz ne peut perturber le résultat de la qualification ;
- cette méthode est très simple, automatique, et ne nécessite aucune qualification particulière et l'intervention d'aucun laboratoire spécialisé;
- le coût de ce contrôle est bien réduit par rapport aux méthodes actuellement pratiquées (à titre d'exemple, un seul analyseur au lieu de cinq analyseurs pour le contrôle de la livraison de N₂, Ar, O₂, CO₂ et du protoxyde d'azote) ;
- selon un des modes avantageux de mise en oeuvre de l'invention, le dispositif en question, préférentiellement "waterproof' i.e de construction imperméable, est positionné « à demeure » dans la zone de livraison des fluides gaziers du site industriel considéré.

La présente invention concerne alors une méthode de contrôle sur site de la qualité des gaz livrés sur un site industriel consommateur, contrôle effectué lors de la livraison, pour déclarer conforme ou non à un cahier des charges la livraison, se caractérisant en ce que l'on effectue, lors de la livraison, un contrôle du ou des gaz livré(s) par la technique de la conductivité thermique, de la façon suivante :
- on dispose lors de la livraison, d'un dispositif d'identification du gaz lors de sa livraison et de sa réception sur le site consommateur, qui comprend :
- un analyseur de la conductivité thermique des gaz, analyseur qui aura été au préalable calibré à l'aide de chacun des gaz purs susceptibles d'être ultérieurement livrés, seuls ou en mélanges, durant une telle livraison ("calibration") ; et
- un système d'acquisition et de traitement de données,
dispositif d'identification du gaz qui est apte :
- à acquérir un ou des échantillons du gaz ou mélange livré ;
- à acquérir un ou des échantillons d'un gaz de référence tel l'azote, gaz de référence préféré puisqu'il est couramment présent sur de tels sites ;
- à effectuer une ou plusieurs mesures de la conductivité du ou des échantillons du gaz de référence et à effectuer la comparaison des mesures obtenues avec les valeurs de conductivité thermique obtenues en amont lors de ladite « calibration" avec ce même gaz de référence, où si lors de cette comparaison, une variation de conductivité est observée, au-delà d'une certaine déviation acceptable fixée au préalable, le dispositif est automatiquement re-calibré, tandis que si le résultat est situé dans la gamme de déviation acceptable le gaz de référence est considéré comme « reconnu » et le dispositif comme « prêt pour opération » ;
- à effectuer une ou plusieurs mesures de la conductivité du ou des échantillons du gaz ou mélange livré, et à effectuer la comparaison des mesures ainsi obtenues avec les valeurs de conductivité thermique obtenues en amont lors de ladite « calibration », où si lors de cette comparaison, le résultat est situé dans une gamme de tolérance acceptable fixée au préalable, le gaz ou mélange livré est considéré comme "reconnu" et la livraison est déclarée "conforme", tandis que si le résultat n'est pas situé dans la gamme de tolérance acceptable la livraison est déclarée « non conforme », le dispositif étant alors apte à donner un diagnostic « livraison conforme » ou « livraison non conforme » autorisant la livraison ou non du gaz ou mélange livré.

Détaillons dans ce qui suit un exemple de mise en oeuvre de l'invention.

Considérons le cas où les livraisons pourront concerner les 3 gaz purs suivants : azote (N₂), oxygène (O₂), et dioxyde de carbone (CO₂).

L'identification selon l'invention se base donc sur la mesure de la conductivité thermique des gaz livrés, qui comme bien connu de la littérature, varie, à une température donnée, pour chaque gaz de façon notable, et permet dès lors d'identifier sans ambiguité le gaz présent.

L'analyseur utilisé a été au préalable calibré, avec des gaz dits "purs", seuls ou en mélanges, dans différentes gammes de teneurs, par exemple pour des mélanges CO₂-O₂, et des mélanges N₂ / O₂.

Le dispositif utilise deux gammes pour les mesures : une première gamme située entre le CO₂ et l'oxygène, et une seconde gamme (pour une meilleure précision) située entre l'azote et l'oxygène.

La partie basse de la gamme est calibrée à "0", tandis que la partie haute de la gamme est calibrée à « 10⁶ », en d'autres termes chaque gamme est calibrée entre 0 et 1 million de points (ppm).

Avec une fréquence prédéfinie, par exemple toutes les 3 heures, le dispositif contrôle de façon automatique le gaz référence en terme de débit et d'identité.

Si une variation de conductivité est observée (au-delà d'une certaine déviation acceptable fixée au préalable), le dispositif est automatiquement re-calibré, tandis que si le résultat est situé dans la gamme de tolérance le gaz de référence est considéré comme « reconnu » et l'équipement comme « prêt pour opération ».

Le système utilise par exemple un critère d'acceptabilité de +/- 10000 points pour identifier un gaz à la valeur cible de la gamme correspondante (voir exemple du tableau 1 ci-dessous).

**Tableau 1**

| Gaz | Valeur cible | Critère d'acceptabilité |
|---|---|---|
| azote | 0 | -10000 à +10000 |
| oxygène | 1 000 000 | 990000 à 1010000 |
| C02 | 0 | -10000 à +10000 |

Une fois le gaz référence "contrôlé", on procède à l'analyse d'au moins un échantillon de gaz livré par le camion sur site, prenons l'exemple de l'oxygène (tableau 1), ici encore avec une valeur cible attendue (10⁶ ppm) et en s'assurant que le résultat est situé dans la gamme de tolérance acceptée.

Si c'est bien le cas, le gaz est considéré comme "reconnu" et la livraison déclarée "conforme", le site livré autorisera donc le transfert du gaz dans ses stockages, tandis que si l'identification n'est pas conforme, la livraison sera refusée.

Le dispositif d'identification est muni d'un profile de configuration qui comprend notamment les paramètres suivants :
- les gaz à identifier (par exemple N₂, O₂, CO₂).
- les valeurs cibles dans chaque cas et le critère (donc la marge) d'acceptabilité.
- le critère de re-calibration (donc la déviation maximum que l'on autorise).
- le nombre de cycles de test à pratiquer (débit, pression, identité).
- les gammes de débit et pression.
- le nombre de cycles de purge et les temps de purge de la ligne de prélèvement d'échantillons.

Un protocole de test est en conséquence fixé à l'avance, par exemple selon les étapes suivantes :
- la ligne de prélèvement d'un échantillon de gaz est connectée au dispositif, avec par exemple une pression d'au moins 1.5 bars, l'opération de mesure est démarrée : en premier lieu le gaz de référence est contrôlé (débit et identité), si les résultats sont situés dans les limites autorisées on en conclu que le dispositif est opérationnel (qualifié) i.e apte à contrôler un ou des échantillons pour l'identification du gaz livré ;
- l'opérateur est alors par exemple interrogé pour confirmer la bonne connexion du dispositif avec la ligne de prélèvement d'un échantillon de gaz ;
- cette ligne de prélèvement de gaz est alors purgée, puis la pression, débit et identité du gaz prélevé sont vérifiés (le nombre de mesures et le nombre de valeurs hors cadre autorisées sont spécifiées dans le profil de configuration de l'analyseur préalablement défini) et l'unité fournit alors un diagnostic d'identité du gaz « conforme » ou « non conforme », information qui peut être stockée (archivée) par l'utilisateur.

De façon générale l'activité du dispositif d'identification peut être archivée dans un fichier, notamment pour pouvoir générer sur demande du site livré un « rapport d'identification ».

## Revendications

1. Méthode de contrôle sur site de la qualité des gaz livrés sur un site consommateur, contrôle effectué lors de la livraison, pour déclarer conforme ou non à un cahier des charges la livraison, **se caractérisant en ce que** l'on effectue, lors de la livraison, un contrôle du ou des gaz livré(s) par la technique de la conductivité thermique, de la façon suivante :
- on dispose lors de la livraison, d'un dispositif d'identification du gaz lors de sa livraison et de sa réception sur le site consommateur, qui comprend :
- un analyseur de la conductivité thermique des gaz, analyseur qui aura été au préalable calibré à l'aide de chacun des gaz purs susceptibles d'être ultérieurement livrés, seuls ou en mélanges, durant une telle livraison ("calibration") ; et
- un système d'acquisition et de traitement de données,
- le dispositif d'identification du gaz est apte :
- à acquérir un ou des échantillons du gaz ou mélange livré ;
- à acquérir un ou des échantillons d'un gaz de référence tel l'azote, gaz de référence préféré puisqu'il est couramment présent sur de tels sites ;
- à effectuer une ou plusieurs mesures de la conductivité du ou des échantillons du gaz de référence et à effectuer la comparaison des mesures obtenues avec les valeurs de conductivité thermique obtenues en amont lors de ladite « calibration » avec ce même gaz de référence, où si lors de cette comparaison, une variation de conductivité est observée, au-delà d'une certaine déviation acceptable fixée au préalable, le dispositif est automatiquement re-calibré, tandis que si le résultat est situé dans la gamme de déviation acceptable le gaz de référence est considéré comme « reconnu » et le dispositif comme « prêt pour opération » ;
- à effectuer une ou plusieurs mesures de la conductivité du ou des échantillons du gaz ou mélange livré, et à effectuer la comparaison des mesures ainsi obtenues avec les valeurs de conductivité thermique obtenues en amont lors de ladite « calibration », où si lors de cette comparaison, le résultat est situé dans une gamme de tolérance acceptable fixée au préalable, le gaz ou mélange livré est considéré comme "reconnu" et la livraison est déclarée "conforme", tandis que si le résultat n'est pas situé dans la gamme de tolérance acceptable la livraison est déclarée « non conforme », le dispositif étant alors apte à donner un diagnostic « livraison conforme » ou « livraison non conforme » autorisant la livraison ou non du gaz ou mélange livré.

2. Méthode selon la revendication 1, **se caractérisant en ce que** le dispositif d'identification est muni d'un profile de configuration qui comprend notamment les paramètres suivants :
- les gaz à identifier, par exemple N₂, O₂, CO₂.
- les valeurs cibles dans chaque cas et la marge d'acceptabilité.
- le critère de re-calibration i.e ladite gamme de tolérance acceptée.
- un nombre de cycles de test à pratiquer.
- des gammes de débit et pression.
- un nombre de cycles de purge et des temps de purge d'une ligne de prélèvement d'échantillons amenant les échantillons prélevés au dispositif.

## Patentansprüche

1. Verfahren zur Vor-Ort-Prüfung der Qualität der an einen Verbraucherstandort gelieferten Gase, wobei die Prüfung bei der Lieferung erfolgt, um anzugeben, ob sie mit einer Produktspezifikation konform sind oder nicht, **dadurch gekennzeichnet, dass** bei der Lieferung eine Prüfung des (der) gelieferten Gase(s) durch die Wärmeleitungstechnik wie folgt durchgeführt wird:
- bei der Lieferung wird eine Vorrichtung zur Identifizierung des Gases bei seiner Lieferung und bei seinem Empfang am Verbraucherstandort angeordnet, die Folgendes umfasst:
- ein Gerät zur Analyse der Wärmeleitfähigkeit der Gase, wobei das Analysegerät zuvor mit jedem der reinen Gase kalibriert worden war, die später allein oder als Gemische während einer solchen Lieferung geliefert werden können ("Kalibrierung); und
- ein System zur Erfassung und Verarbeitung von Daten,
- wobei die Vorrichtung zur Identifizierung des Gases dazu geeignet ist:
- eine Probe oder Proben des gelieferten Gases oder Gemisches zu erfassen;
- eine Probe oder Proben eines Referenzgases wie Stickstoff zu erfassen, das als Referenzgas bevorzugt ist, weil es an solchen Standorten oft vorhanden ist;
- eine oder mehrere Messungen der Leitfähigkeit der Probe oder der Proben des Referenzgases durchzuführen und den Vergleich der erhaltenen Messungen mit den Wärmeleitfähigkeitswerten durchzuführen, die zuvor bei der "Kalibrierung" mit demselben Referenzgas erhalten worden waren, oder, wenn bei diesem Vergleich eine Leitfähigkeitsabweichung über eine zuvor festgelegte bestimmte annehmbare Abweichung hinaus beobachtet wird, die Vorrichtung automatisch neu kalibriert wird, wohingegen, wenn das Ergebnis im annehmbaren Abweichungsbereich liegt, das Referenzgas als "anerkannt" und die Vorrichtung als "betriebsbereit" betrachtet werden;
- eine oder mehrere Messungen der Leitfähigkeit der Probe oder der Proben des gelieferten Gases oder Gemisches durchzuführen und um den Vergleich der so erhaltenen Messungen mit den Wärmeleitfähigkeitswerten durchzuführen, die zuvor bei der "Kalibrierung" erhalten wurden, oder, wenn bei diesem Vergleich das Ergebnis in einem zuvor festgelegten annehmbaren Toleranzbereich liegt, das gelieferte Gas oder Gemisch als "anerkannt" und die Lieferung als "konform" betrachtet werden, wohingegen, wenn das Ergebnis nicht im annehmbaren Toleranzbereich liegt, die Lieferung als "nicht konform" erklärt wird, wobei die Vorrichtung dann dazu geeignet ist, eine Diagnose "konforme Lieferung" oder "nichtkonforme Lieferung" zu geben, welche die Lieferung des gelieferten Gases oder Gemisches autorisiert oder nicht.

2. Verfahren nach Anspruch 1, **dadurch gekennzeichnet, dass** die Identifizierungsvorrichtung mit einem Konfigurationsprofil versehen ist, das insbesondere die folgenden Parameter umfasst:
- die zu identifizierenden Gase, beispielsweise N₂, O₂, CO₂,
- die jeweiligen Zielwerte und die Akzeptanzspanne,
- die Neukalibrierungskriterien, d. h. den akzeptierten Toleranzbereich,
- eine Zahl von durchzuführenden Testzyklen,
- Durchsatz- und Druckbereiche,
- eine Zahl von Spülzyklen und Spülzeiten einer Probenahmeleitung, welche die entnommenen Proben der Vorrichtung zuführt.

## Claims

1. Method for on-site monitoring of the quality of the gases delivered to a consumer site, the monitoring being carried out during the delivery, in order to declare the delivery compliant or non-compliant with specifications, being **characterized in that**, during the delivery, a monitoring of the gas(es) delivered is carried out by the thermal conductivity technique, in the following manner:
- providing, during the delivery, a device for identifying the gas during the delivery thereof and the receipt thereof at the consumer site, which comprises:
- an analyzer of the thermal conductivity of the gases, which analyzer will have been precalibrated using each of the pure gases capable of subsequently being delivered, alone or as mixtures, during such a delivery ("calibration"); and
- a data acquisition and processing system,
- the device for identifying the gas is capable:
- of acquiring one or more samples of the gas or mixture delivered;
- of acquiring one or more samples of a reference gas such as nitrogen, preferred reference gas since it is commonly present at such sites;
- of carrying out one or more measurements of the conductivity of the sample(s) of the reference gas and of carrying out the comparison of the measurements obtained with the thermal conductivity values obtained upstream during said "calibration" with this same reference gas, where if, during this comparison, a variation of conductivity is observed, beyond a certain acceptable deviation set beforehand, the device is automatically recalibrated, whereas if the result lies within the acceptable deviation range, the reference gas is considered to be "recognized" and the device is considered to be "ready for operation";
- of carrying out one or more measurements of the conductivity of the sample (s) of the gas or mixture delivered, and of carrying out the comparison of the measurements thus obtained with the thermal conductivity values obtained upstream during said "calibration", where if, during this comparison, the result lies within an acceptable tolerance range set beforehand, the gas or mixture delivered is considered to be "recognized" and the delivery is declared to be "compliant", whereas if the result does not lie within the acceptable tolerance range the delivery is declared to be "non-compliant", the device then being capable of giving a diagnosis of "compliant delivery" or "non-compliant delivery" authorizing or not authorizing the delivery of the gas or mixture delivered.

2. Method according to Claim 1, **characterized in that** the identification device is equipped with a configuration profile that comprises in particular the following parameters:
- the gases to be identified, for example N₂, O₂, CO₂,
- the target values in each case and the acceptability margin,
- the recalibration criterion, i.e. said acceptable tolerance range,
- a number of test cycles to be carried out,
- flow rate and pressure ranges,
- a number of purge cycles and purge times of a sampling line transporting the samples taken to the device.
